# EUROPEAN PATENT SPECIFICATION

(11) **EP 2 937 331 B1**
(45) Date of publication and mention of the grant of the patent: **21.06.2017**
(21) Application number: 15164824.3
(22) Date of filing: 23.04.2015
(51) Int. Cl.: C07C 67/10

(54) **A PROCESS FOR PREPARING AN INTERMEDIATE OF VITAMIN B1**
VERFAHREN ZUR HERSTELLUNG EINES ZWISCHENPRODUKTS AUS VITAMIN B1
PROCÉDÉ DE PRÉPARATION D'UN INTERMÉDIAIRE DE VITAMINE B1

(30) Priority: 25.04.2014 CN 201410171438
(43) Date of publication of application: 28.10.2015
(73) Proprietor: DSM IP Assets B.V., 6411 TE Heerlen (NL)
(72) Inventor: Zhao, Li-Zhi, 4303 Kaiseraugst (CH); Cao, Wei-Feng, 4303 Kaiseraugst (CH); Zhu, Zhibin, 4303 Kaiseraugst (CH)
(74) Representative: Kurt, Manfred

(56) References cited:
- ARCHER S ET AL: "AN ATTEMPT TO APPLY LETHAL SYNTHESIS TO THE DESIGN OF CHEMOTHERAPEUTIC AGENTS. ÖFLUORINATED 5BETA-(HYDROXYETHYL)-4-METHYLTHIAZOLES", JOURNAL OF MEDICINAL CHEMISTRY, AMERICAN CHEMICAL SOCIETY, US, vol. 22, no. 3, 1 March 1979 (1979-03-01), pages 306-309, XP000575892, ISSN: 0022-2623, DOI: 10.1021/JM00189A017

## Description

### Technical Field

The invention is related a new process for preparing an intermediate of vitamin B₁. Particularly, the invention is related to a new process for preparing O-acyl haloketone.

### Background of the Invention

O-acyl haloketones are important intermediates in the synthesis of vitamin B₁. SU 979344 A and US2193858 disclose a two-steps process for the preparation of O-acyl chloroketones, which includes hydrolysis and decarboxylation of α-chloro-α-aceto-γ-butyrolactone with sulfuric acid or hydrochloric acid and then acylation of the obtained chloroketone.

DE 3013269 discloses a one-step process for the preparation of O-acyl chloroketone, 1-acetoxy-3-chloro-4-pentanone. In the process, α-chloro-α-aceto-γ-butyrolactone is reacted at 70-100°C in the presence of HCl with 0.8-1.2 molar proportions of water and 1-2 molar proportions of acetic acid, and the final product is isolated by distillation of the reaction mixture. However, the final product is not stable under the reaction condition and thus there are many side products such as 3-chloro-5-hydroxypentan-2-one produced in the process.

In order to overcome the defects of the above process, John M. Herbert *et al.* provided a modified process, in which α-chloro-α-acetyl-γ-butyrolactone was mixed with water, acetic acid and concentrated hydrochloric acid and heated to reflux for 18 h, then cooled to room temperature; acetic anhydride was added, and the mixture was heated to 120°C for a further 6 h, then cooled to room temperature and stirred for a further 72 h, to give 5-acetoxy-3-chloro-2-pentanone. (see John M. Herbert et al., J. Label Compd. Radiopharm 2011, 54, 89-92 and see also Archer et al. in J. of Med. Chem., 1979, vol.22, n°3, p.307-308). However, the modified process also includes two steps and thus has long reaction time. In addition, the process cannot be applied generally to produce various O-acyl haloketones, such as O-formyl chloroketone which is sensitive to water present in the reaction.

Therefore, there is still need of a new process which is simple and can be generally applied to produce various O-acyl haloketones.

### Summary of the Invention

The invention provides a new process for preparing O-acyl haloketone, a compound of formula (I), comprising reacting α-halo-α-acyl-butyrolactone of formula (II) with a compound of formula (III) in the presence of an acid catalyst and an organic solvent: 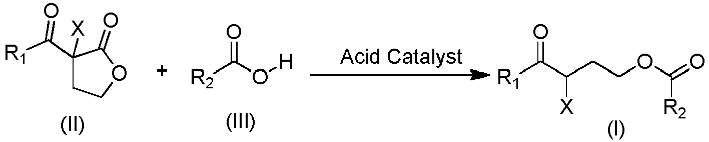 wherein: R₁ is C₁₋₁₀ alkyl or phenyl, R₂ is H or C₁₋₁₀ alkyl, and X is halogen.

### Detailed Description of the Invention

In the present invention, the "C₁₋₁₀ alkyl" refers to linear chain or branched chain, cyclic or non-cyclic saturated alkyl comprising 1-10 carbon atoms. Preferably, the "C₁₋₁₀ alkyl" is C₁₋₄ alkyl, for example but not limited to methyl, ethyl, propyl, isopropyl, cyclopropyl, butyl, isobutyl, tert-butyl, methyl cyclopropyl and cyclobutyl. More preferably, the "C₁₋₁₀ alkyl" is methyl or ethyl.

In the present invention, the "halogen" or "halo" refers to fluoro (F), chloro (Cl), bromo (Br) or iodo (I), preferably chloro (Cl).

The invention provides a process for preparing a compound of the formula (I): 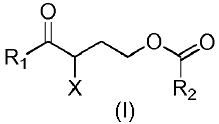 wherein: R₁ is C₁₋₁₀ alkyl or phenyl, R₂ is H or C₁₋₁₀ alkyl, and X is halogen.

Particularly, the invention provides a process for preparing a compound of formula (I), comprising reacting α-halo-α-acyl-butyrolactone of formula (II) with a compound of formula (III) in the presence of an acid catalyst and an organic solvent: 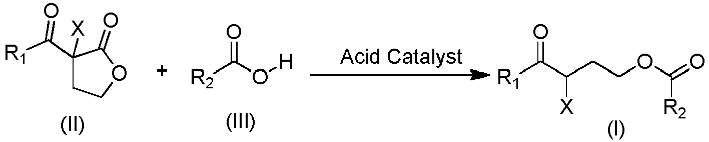 wherein: R₁ is C₁₋₁₀ alkyl or phenyl, R₂ is H or C₁₋₁₀ alkyl, and X is halogen.

The acid catalyst suitable for the reaction may be any strong acid known in the art, for example, those disclosed in SU 979344 A and US2193858. The example of the acid catalyst includes but is not limited to hydrochloric acid, sulfuric acid, p-toluene sulfonic acid (TsOH) and an acidic ion exchange resin, *etc..* Preferably, the acid catalyst is concentrated sulfuric acid or TsOH.

The organic solvent may be any known in the art and suitable for the reaction. Preferably, the organic solvents are those with boiling point of above 80°C. The example of such organic solvent includes but is not limited to toluene, xylene, heptane, cyclohexane, dimethoxyethane (DME), dioxane and mixtures thereof.

In the reaction, the compound of formula (III) may be added in an amount of from 1 mole to 5 moles, preferably from 2 moles to 4 moles, more preferably 3 moles, per mole of α-halo-α-acyl-butyrolactone of the formula (II).

In the reaction, the organic solvent may be added in an amount of from 0.5 L to 2 L, preferably from 0.8 L to 1.5 L, more preferably 1 L, per mole of α-halo-α-acyl-butyrolactone of the formula (II).

The reaction may be carried out at the temperature in a range of from 60°C to 140°C, or from 80°C to 100°C, or preferably under reflux temperature, at an atmospheric pressure.

The process of the present invention is preferably carried out in an inert gas such as a nitrogen environment. The obtained compound of formula (I) can be purified by using known methods such as filtration and evaporation, or used directly without purification into a next reaction, for example, used directly in the synthesis of vitamin B₁.

The process of the present invention provides a new one-step process for preparing O-acyl haloketones. Since the process is carried out in an organic system without water, it is easier to control and has lower side products produced. In addition, the process of the present invention uses a reduced amount of acids and has shorter reaction time compared with the prior processes. Accordingly, the process of the present invention is more appropriate for industrial application.

The present invention is further described by the following examples. These examples are merely for illustrative purposes and should not in any way be interpreted as limiting the scope of the invention.

### Examples

### Example 1: Synthesis of 3-chloro-4-oxopentyl formate 1 using sulfuric acid as the catalyst

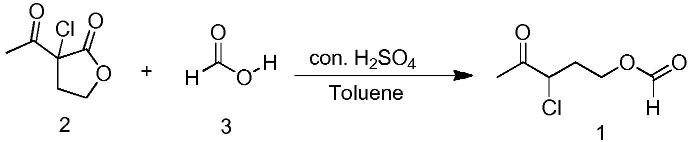

A solution of α-chloro-α-acetyl-butyrolactone 2 (30.2 g, 94.44%, 175 mmol), formic acid 3 (24.7 g, 98%, 526 mmol) and sulfuric acid (95 µL, 98%) in toluene (300 mL) was heated at reflux (90°C) for 19 h under a N₂ gas flow. Then sulfuric acid (48 µL, 98%) was added and the reaction was stirred for additional 3 h. Gas chromatography showed the starting material was mostly consumed. The reaction solution was cooled to the room temperature and filtered to remove the generated dark solid. The filtrate was evaporated to remove the solvent to afford the product 1 (32.1 g) as a dark oil, with 73.6% purity and 82% yield indicated by gas chromatography.
¹H-NMR (400 MHz, DMSO-d6) δ 8.19 (s, 1H), 4.30 - 4.08 (m, 2H), 3.70-3.52 (m, 1H), 3.23 (d, J = 10.1 Hz, 1H), 2.25 (s, 3H), 2.25-2.10 (m, 1H), 1.90-1.75 (m, 1H).

### Example 2: Synthesis of 3-chloro-4-oxopentyl formate 1 using p-toluene sulfonic acid as the catalyst

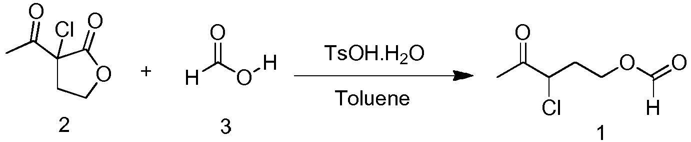

A solution of α-chloro-α-acetyl-butyrolactone 2 (31.46 g, 94.44%, 182.7 mmol), formic acid 3 (26.75 g, 98%, 569.9 mmol) and TsOH•H2O (1.73 g) in toluene (310 mL) was heated at reflux (96°C) for 20 h under a N₂ gas flow. Gas chromatography showed the reaction was complete. The reaction solution was cooled to the room temperature and water (150 mL) was added. The mixture was stirred for 10 mins and the organic phase was separated. The aqueous phase was extracted with toluene (100 mL×2) again. The combined organic phases were washed with brine (100 mL), dried over anhydrous Na₂SO₄, filtered and concentrated to provide the product 1 (31.7 g) as a dark oil, with 82.2% purity and 86.9% yield indicated by gas chromatography.

### Example 3: Synthesis of 3-chloro-4-oxopentyl formate 1 using xylene as the solvent

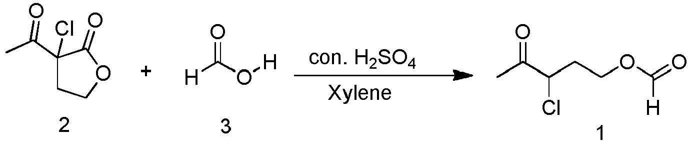

A solution of α-chloro-α-acetyl-butyrolactone 2 (3.08 g, 94.44%, 17.89 mmol), formic acid 3 (2.55 g, 98%, 54.33 mmol) and sulfuric acid (18 mg, 98%) in xylene (15 mL) was heated at 100 °C for 22 h under a N₂ gas flow. Gas chromatography showed the starting material was consumed mostly. The reaction solution was cooled to the room temperature and filtered to remove the generated dark solid. The filtrate was evaporated to afford the product 1 (2.89 g) as a black oil, with 74% purity and 73% yield indicated by gas chromatography.

### Examples 4-6: Synthesis of 3-chloro-4-oxopentyl formate 1 using other solvents

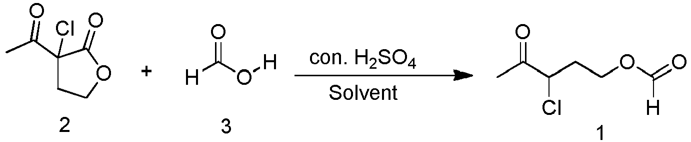

A solution of α-chloro-α-acetyl-butyrolactone 2 (1 g, 94.44%, 5.8 mmol), formic acid 3 (0.82 g, 98%, 17.5 mmol) and sulfuric acid (3 µL, 98%,) in the solvents (10 mL) listed in table below was heated at reflux for 16 h under a N₂ gas flow. Gas chromatography showed the experimental results below.

| Examples | Solvent | Yield of 1 |
|---|---|---|
| 4 | Heptane | 76% |
| 5 | Cyclohexane | 66% |
| 6 | Dimethoxyethane | 45% |

### Example 7: Synthesis of 3-bromo-4-oxopentyl formate 5 using toluene as the solvent

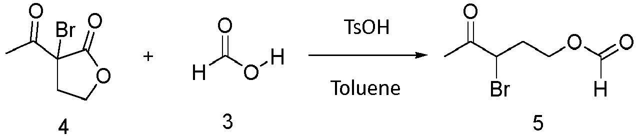

A solution of α-bromo-α-acetyl-butyrolactone 4 (3.63 g, 96.0%, 17.89 mmol), formic acid 3 (2.55 g, 98%, 54.33 mmol) and TsOH (0.17 g, 98%) in toluene (15 mL) was heated at 100°C for 22 h under a N₂ gas flow. Gas chromatography showed the starting material was consumed mostly. The reaction solution was cooled to the room temperature and filtered to remove the generated dark solid. The filtrate was evaporated to afford the product 5 (3.45 g) as a black oil, with 69.3% purity and 68.6% yield indicated by gas chromatography.
¹H-NMR (400 MHz, DMSO-d6) δ 8.05 (s, 1H), 4.45-4.25 (m, 3H), 2.47-2.38 (m, 1H), 2.40 (s, 3H), 2.28-2.19 (m, 1H).

### Example 8: Synthesis of 3-chloro-4-oxopentyl acetic ester 7 using xylene as the solvent

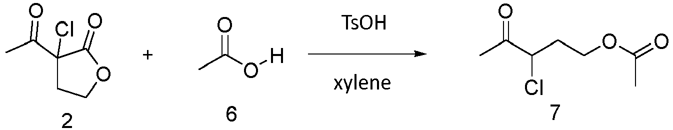

A solution of α-chloro-α-acetyl-butyrolactone 2 (3.08 g, 94.44%, 17.89 mmol), acetic acid 6 (3.33 g, 98%, 54.33 mmol) and TsOH (0.17 g, 98%) in xylene (15 mL) was heated at 140°C for 5 h under a N₂ gas flow. Gas chromatography showed the starting material was consumed mostly. The reaction solution was cooled to the room temperature and filtered to remove the generated dark solid. The filtrate was evaporated to afford the product 7 (3.36 g) as a black oil, with 70.3% purity and 67.8% yield indicated by gas chromatography.
¹H-NMR (400 MHz, DMSO-d6) δ 4.35 (q, 1H), 4.30-4.18 (m, 2H), 2.39-2.31 (m, 4H), 2.16-2.08 (m, 1H), 2.05 (s, 3H).

### Example 9: Synthesis of 3-chloro-4-oxopentyl acetic ester 7 using toluene as the solvent

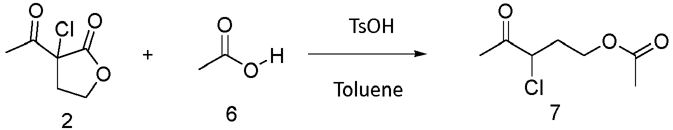

A solution of α-chloro-α-acetyl-butyrolactone 2 (3.08 g, 94.44%, 17.89 mmol), acetic acid 6 (3.33 g, 98%, 54.33 mmol) and TsOH (0.17 g, 98%) in toluene (15 mL) was heated at 140°C for 5 h in a sealed tube. Gas chromatography showed the starting material was consumed mostly. The reaction solution was cooled to the room temperature and filtered to remove the generated dark solid. The filtrate was evaporated to afford the product 7 (3.13 g) as a black oil, with 89.0% purity and 87.2% yield indicated by gas chromatography.

## Claims

1. A process for preparing a compound of formula (I), comprising reacting α-halo-α-acyl-butyrolactone of formula (II) with a compound of formula (III) in the presence of an acid catalyst and an organic solvent: 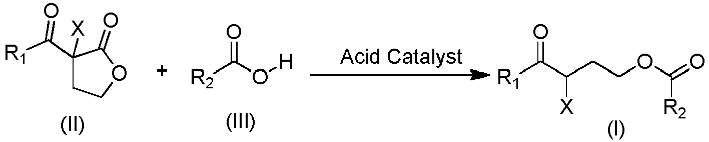 wherein: R₁ is C₁₋₁₀ alkyl or phenyl, R₂ is H or C₁₋₁₀ alkyl, and X is halogen.

2. The process of claim 1, wherein the acid catalyst is selected from the group consisting of hydrochloric acid, sulfuric acid, p-toluene sulfonic acid (TsOH) and an acidic ion exchange resin, preferably, the acid catalyst is concentrated sulfuric acid or TsOH.

3. The process of claim 1, wherein the organic solvent is selected from those with boiling point of above 80°C, such as toluene, xylene, heptane, cyclohexane, dimethoxyethane (DME), dioxane and mixtures thereof.

4. The process of any one of claims 1-3, wherein the compound of formula (III) is added in an amount of from 1 mole to 5 moles, preferably from 2 moles to 4 moles, more preferably 3 moles, per mole of α-halo-α-acyl-butyrolactone of the formula (II).

5. The process of any one of claims 1-3, wherein the organic solvent is added in an amount of from 0.5 L to 2 L, preferably from 0.8 L to 1.5 L, more preferably 1 L, per mole of α-halo-α-acyl-butyrolactone of the formula (II).

6. The process of any one of claims 1-3, wherein the reaction is carried out at the temperature in a range of from 60°C to 140°C, from 80°C to 100°C, preferably, under reflux temperature, at atmospheric pressure.

7. The process of any one of claims 1-3, wherein the reaction is carried out in the absence of water.

## Patentansprüche

1. Verfahren zur Herstellung einer Verbindung der Formel (I), bei dem man ein α-Halogen-α-acylbutyrolacton der Formel (II) in Gegenwart eines Säurekatalysators und eines organischen Lösungsmittels mit einer Verbindung der Formel (III) umsetzt: 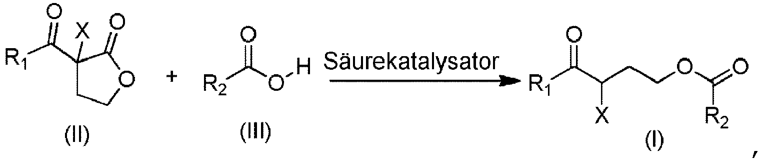 wobei R₁ für C₁₋₁₀-Alkyl oder Phenyl steht, R₂ für H oder C₁₋₁₀-Alkyl steht und X für Halogen steht.

2. Verfahren nach Anspruch 1, bei dem der Säurekatalysator aus der Gruppe bestehend aus Salzsäure, Schwefelsäure, p-Toluolsulfonsäure (TsOH) und einem sauren Ionenaustauscherharz ausgewählt wird und es sich bei dem Säurekatalysator vorzugsweise um konzentrierte Schwefelsäure oder TsOH handelt.

3. Verfahren nach Anspruch 1, bei dem das organische Lösungsmittel aus denjenigen mit einem Siedepunkt über 80°C wie Toluol, Xylol, Heptan, Cyclohexan, Dimethoxyethan (DME), Dioxan und Mischungen davon ausgewählt wird.

4. Verfahren nach einem der Ansprüche 1-3, bei dem die Verbindung der Formel (III) in einer Menge von 1 mol bis 5 mol, vorzugsweise von 2 mol bis 4 mol, weiter bevorzugt 3 mol, pro Mol α-Halogen-α-acylbutyrolacton der Formel (II) zugegeben wird.

5. Verfahren nach einem der Ansprüche 1-3, bei dem das organische Lösungsmittel in einer Menge von 0,5 1 bis 2 l, vorzugsweise von 0,8 l bis 1,5 l, weiter bevorzugt 1 l, pro Mol α-Halogen-α-acylbutyrolacton der Formel (II) zugegeben wird.

6. Verfahren nach einem der Ansprüche 1-3, bei dem die Umsetzung bei einer Temperatur im Bereich von 60°C bis 140°C, von 80°C bis 100°C, vorzugsweise unter Rückflusstemperatur, bei Normaldruck durchgeführt wird.

7. Verfahren nach einem der Ansprüche 1-3, bei dem die Umsetzung in Abwesenheit von Wasser durchgeführt wird.

## Revendications

1. Procédé de synthèse d'un composé de formule (I), comprenant la réaction de l'α-halogéno-α-acyl-butyrolactone de formule (II) avec un composé de formule (III) en présence d'un catalyseur acide et d'un solvant organique : 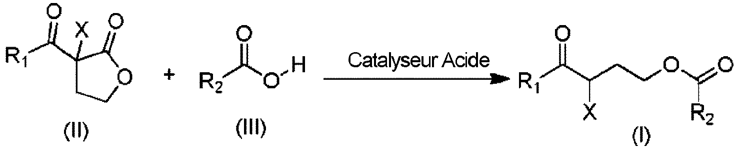 où : R₁ représente un groupement alkyle en C₁₋₁₀ ou phényle, R₂ représente H ou un groupement alkyle en C₁₋₁₀, et X représente un atome d'halogène.

2. Procédé selon la revendication 1, où le catalyseur acide est choisi dans le groupe constitué par l'acide chlorhydrique, l'acide sulfurique, l'acide p-toluènesulfonique (TsOH) et une résine échangeuse d'ions acide, préférentiellement, le catalyseur acide est l'acide sulfurique concentré ou TsOH.

3. Procédé selon la revendication 1, où le solvant organique est choisi parmi ceux dont le point d'ébullition est supérieur à 80 °C, comme le toluène, le xylène, l'heptane, le cyclohexane, le diméthoxyéthane (DME), le dioxane et leurs mélanges.

4. Procédé selon l'une quelconque des revendications 1 à 3, où le composé de formule (III) est ajouté à une teneur comprise entre 1 mole et 5 moles, préférentiellement entre 2 moles et 4 moles, plus préférentiellement 3 moles, par mole d'α-halogéno-α-acyl-butyrolactone de formule (II).

5. Procédé selon l'une quelconque des revendications 1 à 3, où le solvant organique est ajouté à une teneur comprise entre 0,5 L et 2 L, préférentiellement entre 0,8 L et 1,5 L, plus préférentiellement 1 L, par mole d'α-halogéno-α-acyl-butyrolactone de formule (II).

6. Procédé selon l'une quelconque des revendications 1 à 3, où la réaction est mise en oeuvre à une température comprise dans l'intervalle allant de 60 °C à 140 °C, de 80 °C à 100 °C, préférentiellement au reflux, à pression atmosphérique.

7. Procédé selon l'une quelconque des revendications 1 à 3, où la réaction est mise en oeuvre en l'absence d'eau.
